Europäisches Patentamt

European Patent Office

Office européen des brevets

(11) Veröffentlichungsnummer: **0 308 825**
**A1**

(12) **EUROPÄISCHE PATENTANMELDUNG**

(21) Anmeldenummer: 88115216.9

(22) Anmeldetag: 16.09.88

(51) Int. Cl.4: **A61K 7/13 , A61K 7/135 ,**
**C11D 3/39**

(30) Priorität: 24.09.87 DE 3732147

(43) Veröffentlichungstag der Anmeldung:
**29.03.89 Patentblatt 89/13**

(84) Benannte Vertragsstaaten:
**AT BE CH DE ES FR GB GR IT LI LU NL SE**

(71) Anmelder: **Henkel Kommanditgesellschaft auf**
**Aktien**
**Postfach 1100 Henkelstrasse 67**
**D-4000 Düsseldorf 1(DE)**

(72) Erfinder: **Schrader, Dieter**
**Itterstrasse 7**
**D-4000 Düsseldorf(DE)**
Erfinder: **Neuhaus, Winifried**
**Stormstrasse 2**
**D-4020 Mettmann 2(DE)**

(54) **Emulsionsförmige Wasserstoffperoxid-Zubereitungen zum Blondieren und oxidativen Färben der Haare.**

(57) Emulsionsförmige Wasserstoffperoxid-Zubereitungen in Form einer Öl-in-Wasser-Emulsion mit Öl- oder Fettkomponenten, Emulgatoren und Wasserstoffperoxid, die zusätzlich ein in wäßrigem Alkali lösliches, verdik-kendes, carboxylgruppenhaltiges Polymerisat oder Copolymerisat in einer Menge von 1 bis 5 Gew.-% Feststoff, bezogen auf die Zubereitung, enthalten, eignen sich besonders als Oxidationsmittelkomponente in Verfahren zum Färben oder Aufhellen der Haare mit verbesserter Farbtiefe und Brillanz.

EP 0 308 825 A1

## Emulsionsförmige Wasserstoffperoxid-Zubereitungen zum Blondieren und oxidativen Färben der Haare

Die Erfindung betrifft emulsionsförmige Zubereitungen von Wasserstoffperoxid, die sich besonders als Oxidationsmittelzubereitungen zum Blondieren der Haare und zur Färbung der Haare mit Oxidationshaarfärbemitteln eignen.

Das Blondieren und Färben der Haare mit Oxidationshaarfärbemitteln wird in der Regel mit Hilfe von zwei separat verpackten Zubereitungen durchgeführt - der Blondiercreme oder Oxidationshaarfärbecreme (A) und der Oxidationsmittelzubereitung (B) - die erst kurz vor der Anwendung vereinigt und vermischt und dann als gebrauchsfertige Blondier- oder Färbezubereitung auf das Haar gebracht werden. Für die Heimanwendung wurden auch Zweikammer-Misch- und Abgabebehälter entwickelt, in welchen die Komponenten A und B im geeigneten Mengenverhältnis getrennt voneinander abgepackt sind und die eine von außen mechanisch zerstörbare Trennwand zwischen den beiden Kammern aufweisen sowie eine Abgabeöffnung an einer der beiden Kammern. Kurz vor der Anwendung wird die Trennwand zerstört, worauf die Komponenten A und B vereinigt und durch Schütteln oder andere mechanische Maßnahmen vermischt werden. Dann kann die gebrauchsfertige Haarfärbe- oder Blondierzubereitung durch die Abgabeöffnung entnommen bzw. direkt auf das zu färbende Haar aufgetragen werden.

Es hat sich gezeigt, daß es, besonders für die Anwendung in solchen Zweikammer-Misch- und Abgabebehältern vorteilhaft ist, nicht nur die Haarfärbe- oder Blondiercreme (A), sondern auch die Oxidationsmittelzubereitung (B) in Form einer Öl-in-Wasser-Emulsion zu formulieren, da auf diese Weise eine rasche und homogene Mischung mit der Haarfärbe- oder Blondiercreme ermöglicht wird. Als Nachteil dieser in DE-OS 35 34 471 beschriebenen Verfahrensweise hat sich herausgestellt, daß die auf diese Weise erzielbaren Färbungen eine geringere Farbtiefe und Egalität und die erhaltenen Blondierungen weniger Brillanz aufweisen.

Es bestand daher die Aufgabe, diese Nachteile durch geeignete Maßnahmen zu vermeiden. Gegenstand der Erfindung sind Wasserstoffperoxid-Zubereitungen in Form einer Öl-in-Wasser-Emulsion, enthaltend eine Öl- oder Fettkomponente, einen Emulgator und Wasserstoffperoxid, dadurch gekennzeichnet, daß zusätzlich ein in wäßrigem Alkali lösliches, verdickendes carboxylgruppenhaltiges Polymerisat oder Copolymerisat in einer Menge von 1 bis 5 Gew.-% Feststoff, bezogen auf die Zubereitung, enthalten ist.

Als carboxylgruppenhaltiges Polymerisat oder Copolymerisat wird bevorzugt ein Polymerisat oder Copolymerisat der Acrylsäure oder der Methacrylsäure eingesetzt. Solche Polymerisate und Copolymerisate sind an sich bekannt und werden auch als Verdickungsmittel für wäßrige Lösungen seit langem eingesetzt. In GB-A 870 994 werden z. B. Dispersionen von Copolymerisaten aus wenigstens 10 Gew.-% Acrylsäure-Niedrigalkylester, 25 bis 70 Gew.-% Methacrylsäure und ggf. 0 bis 40 Gew.-% eines weiteren Comonomeren mit einem Feststoffgehalt von 25 bis 50 Gew.-% beschrieben. Aus DE-A 11 64 095 sind Mischpolymerisate aus 50 bis 75 Gew.-% Ethylacrylat, 25 bis 35 Gew.-% Acrylsäure und 0 bis 25 Gew.-% anderer Comonomerer bekannt. Diese Copolymerisate können durch Zugabe von vernetzend wirkenden, mehrfach ungesättigten copolymerisierbaren Comonomeren im Molekulargewicht und damit in der Verdickungswirkung modifiziert werden. Besonders wirksame Acrylatdispersionen für diesen Zweck sind z. B. aus DE-A 34 45 549 bekannt.

Die erfindungsgemäß geeigneten Polymerisate stellen meist wäßrige Dispersionen, üblicherweise mit einem Feststoffgehalt von 20 bis 30 Gew.-% dar, die in einem pH-Bereich von etwa 2 bis 5 stabil und dünnflüssig sind. In dem Maße, wie der pH-Wert der verdünnten Lösungen durch wäßrige Basen, z. B. durch Alkalihydroxidlösungen (z. B. Natronlauge oder Kalilauge), Ammoniaklösung, Alkanolamine (z. B. Mono-, Di- oder Triethanolamin) angehoben und die Carboxylgruppen in die Salzform überführt werden, beginnen die Polymerketten sich zu entknäueln und in Lösung zu gehen, wobei sich die Viskosität der Lösung erhöht. Bei einem pH-Wert von ca. 8 ist die völlige Ionisation der Carboxylgruppen und die Viskositätsentwicklung abgeschlossen und klare Wasserlöslichkeit erreicht.

Die erfindungsgemäße Wasserstoffperoxidzubereitung liegt in Form einer Öl-in-Wasser-Emulsion mit einer diskontinuierlichen Öl- oder Fettphase und einer kontinuierlichen, wäßrigen Phase vor. Die Viskosität dieser O/W-Emulsion läßt sich einerseits durch das Verhältnis von Ölphase zu Wasserphase und durch die Art der verwendeten Fettkomponenten in weiten Grenzen steuern. Bevorzugt wird die Viskosität der erfindungsgemäßen Wasserstoffperoxidzubereitung auf 0,5 bis 2 Pa.s, gemessen bei 20 °C und bei einer Scherspannung von D = 3 bis 6 cm$^{-1}$, und auf einen pH-Wert von 3 bis 5 eingestellt.

Als Öl- oder Fettkomponenten können Paraffine, Vaseline, Wachse, Hartfette, kosmetische Ölkomponenten und/oder Fettalkohole eingesetzt werden. Bevorzugt sind in den erfindungsgemäßen Wasserstoffperoxidzubereitungen gesättigte Fettalkohole mit 12 bis 22 C-Atomen als Fettkomponente enthalten.

Als Emulgatoren können anionische, zwitterionische und nichtionogene Tenside enthalten sein. Geeignete anionische Tenside sind gekennzeichnet durch eine bevorzugt lineare Alkylgruppe mit 12 bis 18 C-Atomen und eine daran gebundene anionische Gruppe, z. B. eine -COO$^{(-)}$-, -SO$_3$$^{(-)}$- oder -O-(C$_2$H$_4$O)$_x$-SO$_3$$^{(-)}$-Gruppe, in der x = 0 oder eine Zahl bis 20 bedeutet. Nichtionische Tenside, die sich als Emulgatoren eignen, sind z. B. Anlagerungsprodukte von 2 bis 30 Mol Ethylenoxid an Fettalkohole mit 12 bis 18 C-Atomen, an Alkylphenole mit 8 bis 15 C-Atomen in der Alkylgruppe, an Fettsäuren mit 12 bis 18 C-Atomen, an Glycerin-Fettsäure-(C$_{12}$-C$_{18}$)-partialester, an Sorbitan-Fettsäure-(C$_{12}$-C$_{18}$)-partialester, an Fettamine mit 12 bis 18 C-Atomen, an Fettsäure-(C$_{12}$-C$_{18}$)-monoethanolamid oder an Alkyl-(C$_{12}$-C$_{18}$)-glucoside.

Geeignete zwitterionische Tenside sind z. B. N-C$_{12}$-C$_{18}$-Alkyl-, N,N-dimethylammonio-glycinat, N-C$_{12}$-C$_{18}$-Acylaminopropyl-N,N-dimethylammonio-glycinat oder 2-(N-C$_{12}$-C$_8$-Alkyl-N,N-dimethylammonio)-propionat.

In einer bevorzugten Ausführung enthalten die erfindungsgemäßen Wasserstoffperoxid-Zubereitungen

(A) 1 bis 5 Gew.-% eines gesättigten Fettalkohols mit 16 bis 22 C-Atomen als Fettkomponente

(B) 1 bis 10 Gew.-% eines Fettalkoholoxethylats der Formel R$^1$-O(C$_2$H$_4$O)$_n$-H und/oder eines Alkylsulfats oder Alkylethersulfats der Formel R$^1$-O(C$_2$H$_4$O)$_m$-SO$_3$$^{(-)}$M$^{(+)}$, worin R$^1$ eine C$_{12}$-C$_{18}$-Alkylgruppe, n = 2 bis 30, m = 0 oder 1 bis 30 und M$^{(+)}$ ein Alkali-, NH$_4$$^{(+)}$, 1/2 Mg$^{(++)}$ oder ein Mono-, Di- oder Trialkanolammoniumion mit 2 oder 3 C-Atomen in der Alkanolgruppe sind, als Emulgator

(C) 1,5 bis 15 Gew.-% Wasserstoffperoxid

(D) 1 bis 5 Gew.-% eines in wäßrigem Alkali löslichen, verdickenden Acrylsäure- oder Methacrylsäure-Polymerisats oder Copolymerisats

(E) 66 bis 94 Gew.-% Wasser.

Zusätzlich zu den genannten obligatorischen Komponenten können die erfindungsgemäßen Wasserstoffperoxid-Zubereitungen noch weitere Hilfsmittel in untergeordneten Mengen enthalten. Solche Hilfsmittel sind z. B. - Stabilisatoren für das Wasserstoffperoxid, wie z. B. Dipicolinsäure, Chinolinsäure, Polyphosphate oder die aus DE-B 11 07 207 bekannten Acylierungsprodukte der phosphorigen Säure, z. B. die 1-Hydroxyethan-1,1-diphsophonsäure in einer Menge von ca. 0,05 bis 1,5 Gew.-%

- Puffersubstanzen zur Einstellung eines pH-Wertes von 3 bis 5, bevorzugt saures Natriumpyrophosphat (Na$_2$H$_2$P$_2$O$_7$)

- wasserlösliche Proteinderivate, wasserlösliche kationische Polymere oder andere haarkosmetisch wirksame Komponenten

- und gegebenenfalls Duftstoffe.

Beim Vermischen der erfindungsgemäßen Wasserstoffperoxid-Zubereitungen mit einer alkalisch, bevorzugt auf einen pH-Wert von 8 bis 11 eingestellten Oxidationshaarfärbecreme oder Blondiercreme ergibt sich dann durch Mischung der Emulsionen und Auflösung des carboxylgruppenhaltigen Polymeren eine für den Auftrag auf das Haar geeignete Endviskosität der gebrauchsfertigen Haarfärbe- oder Blondierzubereitung.

Die Oxidationshaarfärbecreme sollte dabei ebenfalls eine Öl-in-Wasser-Emulsion mit einem Gehalt an Öl- oder Fettkomponenten, Emulgatoren und Oxidationshaarfarbstoffvorprodukten mit einem pH-Wert von 8 bis 11 sein, deren Viskosität bevorzugt auf 5 bis 20 Pa.s bei 20 °C und eine Scherspannung von 3 bis 6 cm$^{-1}$ eingestellt ist.

Die Blondiercreme weist einen ähnlichen Aufbau und die gleichen pH-Wert- und Viskositätskriterien auf. Sie enthält aber in der Regel keine Oxidationsfarbstoffe, allenfalls kleine Mengen von Oxidationsfarbstoffvorprodukten, die bei der Anwendung eine Kompensation zu starker Gelb- und Rotnuancen bewirken.

Gegenstand der Erfindung ist auch ein Verfahren zum Färben oder Aufhellen der Haare mit einer Haarfärbe- oder Blondiercreme in Form einer Öl-in-Wasser-Emulsion mit einem pH-Wert von 8 bis 11, dadurch gekennzeichnet, daß man die Haarfärbe- oder Blondiercreme (A) mit einer erfindungsgemäßen Wasserstoffperoxid-Zubereitung (B) in einem Gewichtsverhältnis A : B = 3 : 1 bis 1 : 1 vermischt und das dabei gebildete, gebrauchsfertige Haarfärbe- oder Blondiermittel auf das Haar bringt und nach einer Einwirkungszeit von 10 bis 60 Minuten bei Raumtemperatur wieder abspült.

Die erfindungsgemäßen Wasserstoffperoxid-Zubereitungen lassen sich mit geringem Energieaufwand mit emulsionsförmigen Haarfärbecremes oder Blondiercremes homogen vermischen und eignen sich daher auch für die Abfüllung in die weiter oben beschriebenen Zweikammer-Misch- und Abgabebehälter. Die mit den erfindungsgemäßen Wasserstoffperoxid-Zubereitungen erzeugten Haarfärbungen und Blondierungen zeichnen sich durch eine verbesserte Farbtiefe und Brillanz aus.

Die folgenden Beispiele sollen den Erfindungsgegenstand näher erläutern:

3

## Beispiele

1. Erfindungsgemäße Wasserstoffperoxid-Zubereitungen

| | 1 | 2 |
|---|---|---|
| Cetylalkohol | 1,5 | 2 |
| Lauryl-/Myristylalkohol (70:30) + 2 Mol EO | 3,0 | 2,0 |
| Lauryl-/Myristylalkohol-polyglycol(3 EO)ethersulfat, Na-Salz (28%ige Lösung in $H_2O$) | 8,0 | - |
| Lauryl-/Myristylalkohol-polyglycol(30 EO)ethersulfat, Na-Salz (30%ige Lösung in $H_2O$) | - | 9,0 |
| $Na_2H_2P_2O_7$ | 0,03 | 0,03 |
| Dipicolinsäure | 0,1 | 0,1 |
| 1-Hydroxyethan-1,1-diphosphonsäure | 1,5 | 1,5 |
| Latekoll[R]D (Aerylsäure-Methacrylsäure-copolymerisat, 25%ige Dispersion in $H_2O$) | 10,0 | 15,0 |
| Wasserstoffperoxid (50 Gew.-% in $H_2O$) | 24,0 | 12,0 |
| Ammoniak (25 Gew.-% in $H_2O$) | bis pH = 4 | bis pH = 4 |
| Wasser | ad 100 | ad 100 |

Typische Oxidationshaarfärbecreme (1) und Blondierungscreme (2)

| | A | B |
|---|---|---|
| Fettalkoholgemisch $C_{12}$-$C_{18}$ | 8 | 11 |
| Ölsäurediethanolamid | - | 6 |
| Fettalkohol $C_{16}$-$C_{18}$ + 25 EO | 4 | 3 |
| $(NH_4)_2$-$SO_4$ | 0,65 | 1,0 |
| Oxidationsfarbstoffvorprodukte | 1,5 | - |
| $Na_2SO_3$ | 1,0 | - |
| 1-Hydroxyethan-1,1-diphosphonsäure | 0,1 | - |
| Proteinhydrolysat | - | 0,25 |
| Wasser (u. $NH_3$ bis pH = 9,5) | ad 100 | ad 100 |

Zur Färbung der Haare wurde die Oxidationshaarfärbecreme A mit der Wasserstoffperoxidzubereitung 2 im Gewichtsverhältnis A : 2 = 2 : 1 gemischt. Es entstand eine gebrauchsfertige Oxidationsfärbezubereitung. Zur Blondierung der Haare wurde die Blondiercreme B mit der Wasserstoffperoxidzubereitung 1 im Gewichtsverhältnis B : 1 = 1 : 1 gemischt. Es wurde eine gebrauchsfertige Blondierzubereitung erhalten.

## Ansprüche

1. Wasserstoffperoxid-Zubereitungen in Form von Öl-in-Wasser-Emulsionen, enthaltend Öl- oder Fettkomponenten, Emulgatoren und Wasserstoffperoxid, dadurch gekennzeichnet, daß zusätzlich ein im wäßrigem Alkali lösliches, verdickendes, carboxylgruppenhaltiges Polymerisat oder Copolymerisat in einer Menge von 1 bis 5 Gew.-% Feststoff, bezogen auf die gesamte Zubereitung, enthalten ist.

2. Wasserstoffperoxid-Zubereitungen nach Anspruch 1, dadurch gekennzeichnet, daß

(A) 1 bis 5 Gew.-% eines gesättigten Fettalkohols mit 16 bis 22 C-Atomen als Fettkomponente

(B) 1 bis 10 Gew.-% eines Fettalkoholoxethylats der Formel $R^1-O(C_2H_4O)_n-H$ und/oder eines Alkylsulfats- oder Alkylethersulfats der Formel $R^1-O(C_2H_4O)_m-SO_3^{(-)}M^{(+)}$, worin $R^1$ eine $C_{12}-C_{18}$-Alkylgruppe, n = 2 bis 30, m = 0 oder 1 bis 30 und $M^{(+)}$ ein Alkali-, $NH_4^{(+)}$, 1/2 $Mg^{(++)}$ oder ein Mono-, Di- oder Trialkanolammoniumion mit 2 oder 3 C-Atomen in der Alkanolgruppe darstellt, als Emulgator

(C) 1,5 bis 15 Gew.-% Wasserstoffperoxid

(D) 2 bis 4 Gew.-% eines in wäßrigem Alkali löslichen, verdickenden Acrylsäure- oder Methacrylsäure-Polymerisats oder Copolymerisats

(E) 66 bis 94 Gew.-% Wasser
enthalten.

3. Verfahren zum Färben oder Aufhellen der Haare mit einer Haarfärbecreme oder einer Blondiercreme (A) in Form einer Öl-in-Wasser-Emulsion mit einem pH-Wert von 8 bis 10, dadurch gekennzeichnet, daß man die Haarfärbecreme oder die Blondiercreme mit einer Wasserstoffperoxid-Zubereitung (B) nach den Ansprüchen 1 bis 3 in einem Gewichtsverhältnis von A : B = 3 : 1 bis 1 : 1 vermischt und das dabei gebildete, gebrauchsfertige Haarfärbemittel oder Blondiermittel auf das Haar bringt und nach einer Einwirkungszeit von 15 bis 60 Minuten bei Raumtemperatur abspült.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 182 781 (HENKEL) <br> * Insgesamt * <br> --- | 1-3 | A 61 K 7/13 <br> A 61 K 7/135 <br> C 11 D 3/39 |
| Y | FR-A-2 303 075 (SOLVAY) <br> * Insgesamt * <br> --- | 1-3 | |
| D,Y | EP-A-0 216 334 (HENKEL) <br> * Seite 5, oxidationsmittelzubereitung * <br> --- | 1-3 | |
| A | EP-A-0 092 932 (INTEROX CHEMICALS LTD.) <br> * Insgesamt * <br> --- | 1,2 | |
| A | CH-A- 387 211 (UNILEVER) <br> * Beispiel 5 * <br> --- | 1-3 | |
| A | DE-C- 716 197 (KEIL) <br> * Insgesamt * <br> --- | 1-3 | |
| A | DE-A-1 902 261 (L'OREAL) <br> * Insgesamt * <br> ----- | 1-3 | **RECHERCHIERTE SACHGEBIETE (Int. Cl.4)** <br><br> A 61 K <br> A 01 N <br> C 11 D |

Der vorliegende Recherchenbericht wurde für alle Patentansprüche erstellt

| Recherchenort | Abschlußdatum der Recherche | Prüfer |
|---|---|---|
| DEN HAAG | 02-12-1988 | FISCHER J.P. |